# EUROPEAN PATENT SPECIFICATION

(11) **EP 3 332 712 B1**
(45) Date of publication and mention of the grant of the patent: **02.07.2025**
(21) Application number: 17206010.5
(22) Date of filing: 07.12.2017
(51) Int. Cl.: A61B 8/00, A61B 8/08, G06T 7/00, G16H 30/20, G16H 40/63

(54) **GUIDED NAVIGATION OF AN ULTRASOUND PROBE**
GEFÜHRTE NAVIGATION EINER ULTRASCHALLSONDE
NAVIGATION GUIDEE D'UNE SONDE ULTRASON

(30) Priority: 07.12.2016 FR 1662038
(43) Date of publication of application: 13.06.2018
(73) Proprietor: Caption Health, Inc., San Francisco, CA 94132 (US)
(72) Inventor: CADIEU, Charles, SAN FRANCISCO, CA California 94132 (US); HONG, Ha, SAN FRANCISCO, CA California 94132 (US); KOEPSELL, Kilian, SAN FRANCISCO, CA California 94132 (US); MATHE, Johan, SAN FRANCISCO, CA California 94132 (US); WOJTCZYK, Martin, SAN FRANCISCO, CA California 94132 (US)
(74) Representative: Gevers & Orès

(56) References cited:
- EP-A1- 2 807 978
- WO-A1-2015/150932
- US-A1- 2012 035 868
- US-A1- 2016 174 934

## Description

### BACKGROUND OF THE INVENTION

### Field of the Invention

The present invention relates to ultrasound imaging and more particularly to ultrasound image acquisition.

### Description of the Related Art

The documents WO-A1-2015/150932, EP-A1-2 807 978, US-A1-2012/035868 and US-A1-2016/174934 represent the technological background of the invention.

Ultrasound imaging, also known as sonography, is a medical imaging technique that employs high-frequency sound waves to view three-dimensional structures inside the body of a living being. Because ultrasound images are captured in real-time, ultrasound images also show movement of the the internal organs of the body as well as blood flowing through the blood vessels of the human body and the stiffness of tissue. Unlike x-ray imaging, ultrasound imaging does not involve ionizing radiation thereby allowing prolonged usage of ultrasound imaging without threatening tissue and internal organ damage from prolonged radiation exposure.

To acquire ultrasound imagery, during an ultrasound exam, a transducer, commonly referred to as a probe, is placed directly on the skin or inside a body opening. A thin layer of gel is applied to the skin so that the ultrasound waves are transmitted from the transducer through the medium of the gel into the body. The ultrasound image is produced based upon a measurement of the reflection of the ultrasound waves off the body structures. The strength of the ultrasound signal, measured as the amplitude of the detected sound wave reflection, and the time taken for the sound wave to travel through the body provide the information necessary to compute an image.

Compared to other prominent methods of medical imaging, ultrasound presents several advantages to the diagnostician and patient. First and foremost, ultrasound imaging provides images in real-time. As well, ultrasound imaging requires equipment that is portable and can be brought to the bedside of the patient. Further, as a practical matter, the ultrasound imaging equipment is substantially lower in cost than other medical imaging equipment, and as noted, does not use harmful ionizing radiation. Even still, the production of quality ultrasound images remains highly dependent upon a skilled operator.

In this regard, depending upon the portion of the body selected for imaging, the skilled operator must know where to initially place the ultrasound probe. Then, the skilled operator must know how to spatially orient the probe and finally, the skilled operator must know where to move the probe so as to acquire the desired imagery. Generally, the ultrasound operator is guided in the initial placement, orientation and movement of the probe based upon the visual feedback provided by the imagery produced during the ultrasound. Thus, essentially, the navigation of the probe is a manual process consisting of iterative trial and error. Plainly, then, the modern process of ultrasound navigation is not optimal.

### BRIEF SUMMARY OF THE INVENTION

Embodiments of the present invention address deficiencies of the art in respect to ultrasound probe navigation and provide a novel and non-obvious method, system and computer program product for the guided navigation of an ultrasound probe. In an embodiment of the invention, an ultrasound navigation assistance method according to claim 1 is disclosed.

In another embodiment of the invention, an ultrasound imaging data processing system according to claim 7 is disclosed.

Additional aspects of the invention will be set forth in part in the description which follows, and in part will be obvious from the description, or may be learned by practice of the invention. The aspects of the invention will be realized and attained by means of the elements and combinations particularly pointed out in the appended claims. It is to be understood that both the foregoing general description and the following detailed description are exemplary and explanatory only and are not restrictive of the invention, as claimed.

### BRIEF DESCRIPTION OF THE SEVERAL VIEWS OF THE DRAWINGS

The accompanying drawings, which are incorporated in and constitute part of this specification, illustrate embodiments of the invention and together with the description, serve to explain the principles of the invention. The embodiments illustrated herein are presently preferred, it being understood, however, that the invention is not limited to the precise arrangements and instrumentalities shown, wherein:
Figure 1 is a pictorial illustration of a process for guided navigation of an ultrasound probe
Figure 2 is a schematic illustration of an ultrasound data processing system configured for guided navigation of an ultrasound probe; and,
Figure 3 is a flow chart illustrating a process for guided navigation of an ultrasound probe.

### DETAILED DESCRIPTION OF THE INVENTION

Embodiments of the invention provide for guided navigation of an ultrasound probe. In accordance with an embodiment of the invention, an ultrasound probe is placed on the surface of a body. Then, imagery of a target organ of the body is acquired and a deviation of a contemporaneous pose of the ultrasound pose evident from the acquired image from an optimal pose for the target organ is presented to an end user operator of the ultrasound probe. For example, the deviation is presented visually in respect to a corresponding display, audibly by way of an audible guidance signal, or in the alterative, by way of the text to speech presentation of textual instructions, or haptically through the outer shell of the ultrasound probe.

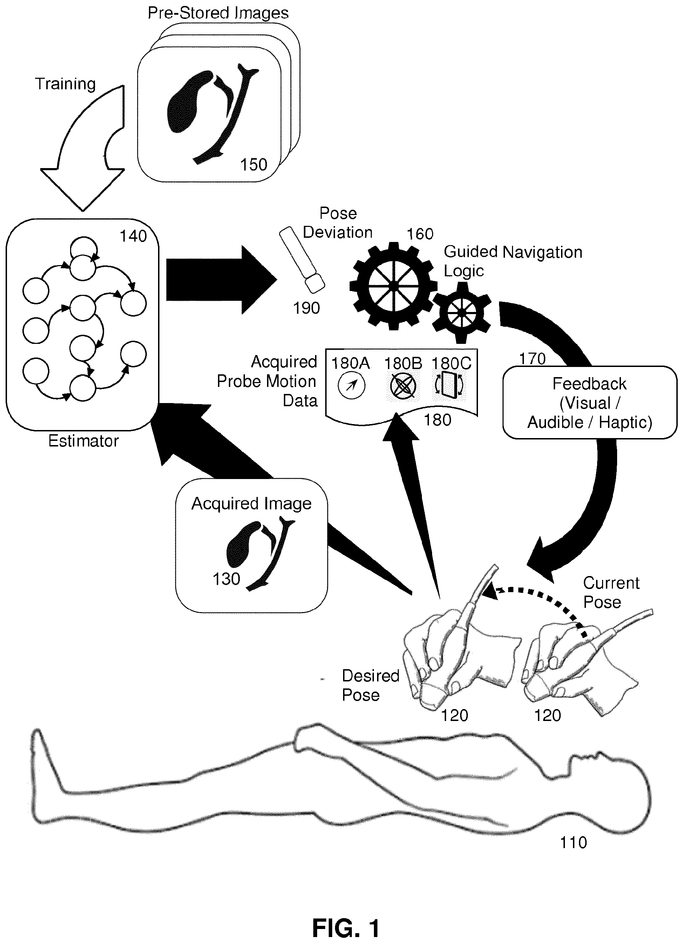

In illustration, Figure 1 is a pictorial illustration of a process for guided navigation of an ultrasound probe. As shown in Figure 1, an ultrasound probe 120 is placed upon an outer surface of a body 110 such as a human form. Imagery 130 of a target organ is acquired by the operator of the ultrasound probe 120 and the image 130 of the target organ is presented input to an estimator 140, such as a neural network. The estimator 140 is trained based upon a set of training images 150 of one or more different target organs, each with a known probe pose deviation from the optimal probe pose so that the input of the contemporaneously acquired image 130 to the estimator 140 produces an output of a deviation 190 of the contemporaneous pose of the ultrasound probe 120 from an optimal pose of the ultrasound probe 120.

Optionally, the ultrasound probe 120 acquires probe orientation and movement data 180 including magnetomic information 180A, gyroscopic information 180B and accelerometric information 180C indicating an orientation and movement of the ultrasound probe 120 so as to compute the change in the pose of the ultrasound probe 120. Guided navigation logic 160 then processes the probe orientation and movement data 180 so as to better compute the deviation from the optimal pose 190 in consideration not only of the pose deviation 190 output by the estimator 140 in respect to the acquired image 130 but also in consideration of the change in the pose of the ultrasound probe 120 determined from the probe orientation and movement data 180.

Guided navigation logic 160 then processes the pose deviation 190 of the ultrasound probe 120 and emits feedback 170 in the form of visual feedback such as a three-dimensionally rendered scene with the two probe models showing current and optimal probe poses with suggested maneuver; the amount of agreement between the current and optimal poses; or red, green or yellow colors indicating how large an adjustment of the orientation of the ultrasound probe 120 is required to approach the optimal pose, audible feedback such as a tone, or haptic feedback. In regard to the latter, in one aspect of the invention the ultrasound probe 120 may be caused to vibrate more intensely or with greater frequency responsive to the pose deviation 190.

In respect to the former, in one aspect of the invention the ultrasound probe 120 may be caused to emit a sound that is more intense of a different tone when the ultrasound probe 120 based upon the magnitude of the pose deviation 190. As well the ultrasound probe 120 may be caused to emit a short-duration sound such as a click or pop repeatedly with a frequency related to the magnitude of the pose deviation 190. Alternatively, in another aspect of the invention the ultrasound probe 120 may be caused to vibrate more intensely or with greater frequency when the ultrasound probe 120 is moved in a compliant manner based upon a magnitude of the pose deviation 190.

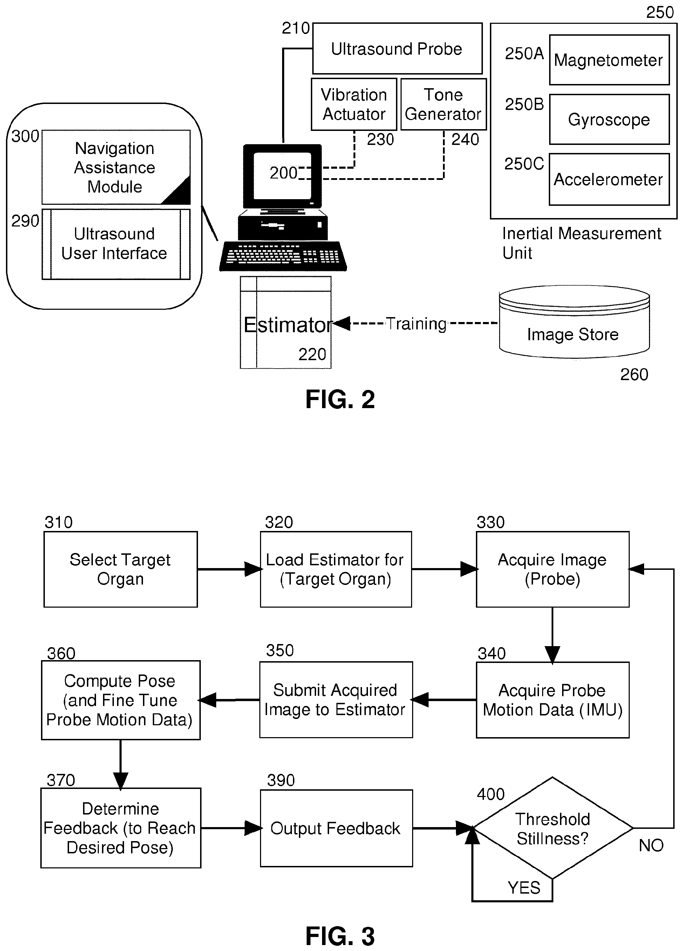

The process described in connection with Figure 1 may be implemented in an ultrasound data processing system. In further illustration, Figure 2 schematically illustrates an ultrasound data processing system configured for guided navigation of an ultrasound probe. The system includes an ultrasound probe 210 coupled to a host computing system 200 of one or more computers, each with memory and at least one processor. The ultrasound probe 210 is enabled to acquire ultrasound imagery by way of a transducer connected to beamformer circuitry in the host computing system 200, and transmit the acquired ultrasound imagery to the beamformer circuitry of the host computing system 200 for display in a display of the host computing system 200 through an ultrasound user interface 290 provided in the memory of the host computing system 200.

The ultrasound probe 210 includes an electromechanical vibration generator 230 such as a piezo actuator, and a tone generator 240. The electromechanical vibration generator 230 may be driven in the ultrasound probe 210 to cause the ultrasound probe 210 to vibrate at a specific frequency and for a specific duration as directed by the host computing system 200. As well, the tone generator 240 may be driven in the ultrasound probe 210 to cause the ultrasound probe 210 to emit an audible tone at a specific frequency and amplitude and for a specific duration as directed by the host computing system 200. Optionally, the tone generator 240 may be disposed in the host computing system 200.

An image data store 260 stores therein a multiplicity of different ultrasound images previously acquired in a controlled setting where the pose deviation of each image is known. The images of the image store 260 are provided as training images in training an estimator 220 such as a neural network providing decisioning of a deviation from an optimal probe pose relative to an input image of a target organ of a human form. In this regard, the estimator 220 includes a multiplicity of nodes processing different extracted features of an acquired image so as to decision a pose of the ultrasound probe providing a deviation of the decisioned pose from a known, optimal pose in imaging a target organ. In this regard, the pose can be represented mathematically in Euclidian space or any array of numbers.

Finally, a navigation assistance module 300 is coupled to the ultrasound user interface 290. The navigation assistance module 300 includes program code that when executed in the memory of the host computing system 200 acquires a contemporaneous ultrasound image by the ultrasound probe 210 and processes the acquired ultrasound image in the host computing platform 200 utilizing the estimator 220. The program code of the navigation assistance module 300 during execution in the memory of the host computing system 200 then receives with the assistance of the estimator 220 a computed deviation of a pose evident from the acquired image, from an optimal pose of the ultrasound probe 210.

Optionally, the ultrasound probe 210 includes an inertial measurement unit 250. The inertial measurement unit 250 includes each of a magnetometer 250A, a gyroscope 250B, and an accelerometer 250C. As such, data acquired by the inertial measurement unit 250 is translated in the host computing system 200 to estimate a change in pose for a given time interval, for instance by measuring linear acceleration and angular velocity of the probe 210. This change in pose can be combined with an estimator-derived pose deviation to obtain a more precise pose estimate. One possible example includes the use of a Kalman filter.

For instance, algorithmically, the process utilizing the inertial measurement unit 250 to tune a determined deviation from the estimator 220 can be expressed as follows:
1. Let ***I***(*t*) be the image acquired by the ultrasound probe 210 at time *t.*
2. Let *f* be an estimator such as neural network 220 that from an acquired image ***I***(*t*) outputs the image-estimated pose ***p***(*t*)_{img} at time *t* relative to the optimal pose (i.e., deviation of the pose from the optimal pose). That is, ***p***(*t*)_{img} = *f*(***I***(*t*))*.*
3. Between *t*₀ and *t*₁ (> *t*₀), the change of pose Δ***p***(*t*₁, *t*₀)_{img} can be computed as: Δ***p***(*t*₁, *t*₀)_{img} *= **p***(*t*₁)_{img} *- **p***(*t*₀)_{img}*.* Obviously,***p***(*t*₁)_{img} = ***p***(*t*₀)_{img} + Δ***p***(*t*₁, *t*₀)_{img}.
4. The change of pose between *t*₀ and *t*₁ is measured from the inertial measurement unit 250 and denoted as Δ***p***(*t*₁, *t*₀)_{IMU}. Δ***p***(*t*₁, *t*₀)_{img} and Δ***p***(*t*₁, *t*₀)_{IMU} are combined to produce a better estimate of the pose change by using a Kalman filter expressed as Δ***p***(*t*₁, *t*₀)*_{K} = K*(Δ***p***(*t*₁, *t*₀)_{img}, Δ***p***(*t*₁, *t*₀)_{IMU}; ***p***(*t*₀)_{img}) where K is a Kalman filter (that takes image-based pose change, inertial measurement unit 250 based pose change, and the pose at *t*₀ as inputs), Δ***p***(*t*₁, *t*₀)*_{K}* is the combined pose change that is expected to be more accurate than either Δ***p***(*t*₁, *t*₀)_{img} or Δ***p***(*t*₁, *t*₀)_{IMU} alone.
5. With the more accurate Δ***p***(*t*₁, *t*₀)*_{K}*, it is then possible to estimate more accurate absolute pose ***p***(*t*₁)*_{K}* at time *t*₁: ***p***(*t*₁)*_{K} = **p***(*t*₀)_{img} + Δ***p***(*t*₁, *t*₀)*_{K}*
6. For all subsequent time points: ***p***(*t*_{*j +* 1})*_{K} = **p***(*tⱼ*)*_{K} +* Δ***p***(*tⱼ*, *t*_{*j +* 1})*_{K}*

In any event, based upon the computed deviation, the program code of the navigation assistance module 300 then determines corresponding feedback to be presented through the ultrasound probe 210. For example, the program code of the navigation assistance module 300 may direct the tone generator 240 to emit a particular tone pattern of specific periodicity proportional or inversely proportional to a determined proximity of the ultrasound probe 210 to the optimal pose. As another example, the program code of the navigation assistance module 300 may direct the electromechanical vibration generator 230 of the ultrasound probe 210 to emit a particular vibration of specific intensity proportional or inversely proportional to a determined proximity of the ultrasound probe 210 to the optimal pose.

In yet further illustration of the operation of the navigation assistance module 300, Figure 3 is a flow chart depicting a process for guided navigation of an ultrasound probe. Beginning in block 310, a target organ within the body is selected in a user interface to an ultrasound application visualizing ultrasound imagery acquired by the ultrasound probe. Subsequently, in block 320 an estimator such as a neural network pertaining to the target organ is loaded into memory of a computing system coupled to the ultrasound probe. In block 330, a contemporaneous ultrasound imagery is acquired by the ultrasound probe and in block 340, optionally, probe orientation and movement data is received from an inertial measurement unit of the ultrasound probe is acquired. In block 350, the contemporaneous ultrasound imagery is processed in connection with to the estimator in the computing system.

In block 360, a deviation of a pose of the ultrasound probe from an optimal pose that is evident from the acquired image is determined based upon the application of the estimator to the acquired image. Optionally, the probe orientation and movement data are used to further improve the accuracy of the determined pose deviation. In block 370, corresponding feedback based upon the deviation is determined such as a graphical representation of the deviation, a particular strength of vibration as part of haptic feedback, or a particular tone of particular frequency, periodicity, amplitude or any combination thereof as part of audible feedback. In block 390, then, the determined feedback is output by the computing system or the coupled ultrasound probe. Finally, in decision block 400, the inertial measurement unit of the ultrasound probe indicates whether or not a threshold change in position or orientation has occurred with respect to the ultrasound probe. If so, or in case where an inertial measurement unit is not present or active, the process may then repeat through block 340.

The present invention may be embodied within a system, a method, a computer program product or any combination thereof. The computer program product may include a computer readable storage medium or media having computer readable program instructions thereon for causing a processor to carry out aspects of the present invention. The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Computer readable program instructions described herein can be downloaded to respective computing/processing devices from a computer readable storage medium or to an external computer or external storage device via a network. The computer readable program instructions may execute entirely on the user's computer, partly on the user's computer, as a stand-alone software package, partly on the user's computer and partly on a remote computer or entirely on the remote computer or server. Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Finally, the terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

Having thus described the invention of the present application in detail and by reference to embodiments thereof, it will be apparent that modifications and variations are possible without departing from the scope of the invention defined in the appended claims as follows:

## Claims

1. An ultrasound navigation assistance method comprising:
acquiring an image (130) by an ultrasound probe (120; 210) of a target organ of a body (110) (330);
processing the image in connection with an estimator (140; 220) comprising a neural network, the estimator producing a deviation (190) of a contemporaneous pose indicative of an orientation of the ultrasound probe evident from the image from an optimal pose of the ultrasound probe for imaging the target organ (350);
wherein a change of pose obtained with an inertial measurement unit (250) is combined with a deviation produced by the estimator (140, 220) using a Kalman filter, to tune the deviation produced by the estimator (140; 220) and,
presenting the produced deviation to an end user operator of the ultrasound probe.

2. The method of claim 1, wherein probe orientation and movement data (180) is received from the inertial measurement unit (250) comprising at least one of an accelerometer (250C), gyroscope (250B) and magnetometer (250A).

3. The method of claim 1, wherein the produced deviation (190) is presented visually in a display of a computer system coupled to the probe (120; 210).

4. The method of claim 1, wherein the produced deviation (190) is presented audibly through a varying of a tone based upon a proximity of the probe (120; 210) to the optimal pose.

5. The method of claim 1, wherein the produced deviation (190) is presented audibly by varying a frequency of repeatedly audibly presenting a short-duration sound based upon a proximity of the probe (120; 210) to the optimal pose.

6. The method of claim 1, wherein the produced deviation (190) is presented haptically through a varying of vibrations of the probe based upon a proximity of the probe (120; 210) to the optimal pose.

7. An ultrasound imaging data processing system configured for ultrasound navigation assistance, the system comprising:
a computer with memory and at least one processor;
a display coupled to the computer;
beamformer circuitry coupled to the computer and the display;
an ultrasound probe (120; 210) comprising a transducer connected to the beamformer circuitry; and,
a navigation assistance module (300) executing in the memory of the computer, the module comprising program code enabled upon execution by the processor of the computer to acquire an image (130) by the ultrasound probe of a target organ of a body (110), to process with an estimator (140; 220) comprising a neural network, the acquired image by determining a deviation (190) between a contemporaneous pose of the ultrasound probe relative to the target organ evident from the acquired image, and an optimal pose of the ultrasound probe for imaging the target organ,
wherein a change of pose obtained with an inertial measurement unit (250) is combined with the deviation produced by the estimator (140; 220) using a Kalman filter,
to tune the deviation produced by the estimator (140; 220); and
to present the determined deviation to an end user operator of the ultrasound probe.

8. The system of claim 7, wherein probe orientation and movement data (180) is received from the inertial measurement unit (250) comprising at least one of an accelerometer (250C), gyroscope (250B) and magnetometer (250A).

9. The system of claim 7, wherein the determined deviation (190) is presented visually in a display of a computer system coupled to the probe (120, 210).

10. The system of claim 7, wherein the determined deviation (190) is presented audibly through a varying of a tone based upon a proximity of the probe (120; 210) to the optimal pose.

11. The system of claim 7, wherein the determined deviation (190) is presented haptically through a varying of vibrations of the probe (120; 210) based upon a proximity of the probe to the optimal pose.

12. A computer program product for ultrasound navigation assistance, the computer program product comprising a computer readable storage medium having program instructions embodied therewith, the program instructions executable by a device to cause the device to perform a method comprising:
acquiring an image (130) by an ultrasound probe (120; 210) of a target organ of a body (110);
processing the image in connection with an estimator (140; 220) comprising a neural network, the estimator producing a deviation (190) of a contemporaneous pose indicative of an orientation of the ultrasound probe evident from the image from an optimal pose of the ultrasound probe for imaging the target organ (350);
wherein a change of pose obtained with an inertial measurement unit (250) is combined with the deviation obtained with the estimator (140; 220) using a Kalman filter,
to tune the deviation produced by the estimator (140; 220) and,
presenting the produced deviation to an end user operator of the ultrasound probe.

13. The computer program product of claim 12, wherein probe orientation and movement data (180) is received from an inertial measurement unit (250) comprising at least one of an accelerometer (250C), gyroscope (250B) and magnetometer (250A).

14. The computer program product of claim 12, wherein the produced deviation (190) is presented visually in a display of a computer system coupled to the probe (120; 210).

15. The computer program product of claim 12, wherein the produced deviation (190) is presented audibly through a varying of a tone based upon a proximity of the probe (120; 210) to the optimal pose.

16. The computer program product of claim 12, wherein the produced deviation (190) is presented audibly by varying a frequency of repeatedly audibly presenting a short-duration sound based upon a proximity of the probe (120; 210) to the optimal pose.

17. The computer program product of claim 12, wherein the produced deviation is presented haptically through a varying of vibrations of the probe (120; 210) based upon a proximity of the probe to the optimal pose.

## Patentansprüche

1. Ultraschall-Navigationsunterstützungsverfahren, umfassend:
Erheben eines Bildes (130) eines Zielorgans eines Körpers (110) durch eine Ultraschallsonde (120; 210) (330);
Verarbeiten des Bildes in Verbindung mit einem Schätzer (140, 220), der ein neuronales Netzwerk umfasst, wobei der Schätzer eine Abweichung (190) einer zeitgleichen Stellung, die eine aus dem Bild ersichtliche Ausrichtung der Ultraschallsonde angibt, von einer für die Bildgebung des Zielorgans (350) optimalen Stellung der Ultraschallsonde erzeugt;
wobei eine Stellungsänderung, die mit einer Trägheitsmesseinheit (250) erhalten wird, mit einer von den Schätzer (140, 220) erzeugten Abweichung unter Verwendung eines Kalman-Filters kombiniert wird, um die von dem Schätzer (140; 220) erzeugte Abweichung feinabzustimmen, und
Präsentieren der erzeugten Abweichung für einen Endbenutzer-Bediener der Ultraschallsonde.

2. Verfahren nach Anspruch 1, wobei Sondenausrichtungs- und -bewegungsdaten (180) von der Trägheitsmesseinheit (250) empfangen werden, die mindestens eines von einem Beschleunigungsmesser (250C), einem Gyroskop (250B) und einem Magnetometer (250A) umfasst.

3. Verfahren nach Anspruch 1, wobei die erzeugte Abweichung (190) visuell auf einer Anzeige eines Computersystems, das mit der Sonde (120; 210) gekoppelt ist, präsentiert wird.

4. Verfahren nach Anspruch 1, wobei die erzeugte Abweichung (190) hörbar durch ein Variieren eines Tons basierend auf einer Nähe der Sonde (120; 210) zu der optimalen Stellung präsentiert wird.

5. Verfahren nach Anspruch 1, wobei die erzeugte Abweichung (190) hörbar durch Variieren einer Frequenz einer wiederholten hörbaren Präsentation eines kurzen Tons basierend auf einer Nähe der Sonde (120; 210) zu der optimalen Stellung präsentiert wird.

6. Verfahren nach Anspruch 1, wobei die erzeugte Abweichung (190) haptisch durch ein Variieren von Vibrationen der Sonde basierend auf einer Nähe der Sonde (120; 210) zu der optimalen Stellung präsentiert wird.

7. Ultraschallbildgebungs-Datenverarbeitungssystem, das zur Ultraschall-Navigationsunterstützung konfiguriert ist, wobei das System Folgendes umfasst:
einen Computer mit Speicher und mindestens einem Prozessor;
eine Anzeige, die mit dem Computer gekoppelt ist;
Strahlformerschaltungsanordnung, die mit dem Computer und der Anzeige gekoppelt sind;
eine Ultraschallsonde (120; 210), die einen Wandler umfasst, der mit der Strahlformerschaltungsanordnung verbunden ist; und
ein Navigationsunterstützungsmodul (300), das im Speicher des Computers ausgeführt wird, wobei das Modul Programmcode umfasst, der bei Ausführung durch den Prozessor des Computers aktiviert wird, um über die Ultraschallsonde ein Bild (130) eines Zielorgans eines Körpers (110) zu erheben, das erhobene Bild mit einem Schätzer (140; 220), der ein neuronales Netzwerk umfasst, durch Bestimmen einer Abweichung (190) zwischen einer zeitgleichen Stellung der Ultraschallsonde relativ zu dem Zielorgan, die aus dem erhobenen Bild ersichtlich ist, und einer optimalen Stellung der Ultraschallsonde für die Bildgebung des Zielorgans zu verarbeiten,
wobei eine Stellungsänderung, die mit einer Trägheitsmesseinheit (250) erhalten wird, mit der von dem Schätzer (140, 220) erzeugten Abweichung unter Verwendung eines Kalman-Filters kombiniert wird, um die von dem Schätzer (140; 220) erzeugte Abweichung feinabzustimmen; und die bestimmte Abweichung für einen Endbenutzer-Bediener der Ultraschallsonde zu präsentieren.

8. System nach Anspruch 7, wobei Sondenausrichtungs- und -bewegungsdaten (180) von der Trägheitsmesseinheit (250) empfangen werden, die mindestens eines von einem Beschleunigungsmesser (250C), einem Gyroskop (250B) und einem Magnetometer (250A) umfasst.

9. System nach Anspruch 7, wobei die bestimmte Abweichung (190) visuell auf einer Anzeige eines mit der Sonde (120, 210) gekoppelten Computersystems präsentiert wird.

10. System nach Anspruch 7, wobei die bestimmte Abweichung (190) hörbar durch ein Variieren eines Tons basierend auf einer Nähe der Sonde (120; 210) zu der optimalen Stellung präsentiert wird.

11. System nach Anspruch 7, wobei die bestimmte Abweichung (190) haptisch durch ein Variieren von Vibrationen der Sonde (120; 210) basierend auf einer Nähe der Sonde zu der optimalen Stellung präsentiert wird.

12. Computerprogrammprodukt zur Ultraschall-Navigationsunterstützung, wobei das Computerprogrammprodukt ein computerlesbares Speichermedium umfasst, das Programmanweisungen damit verkörpert aufweist, wobei die Programmanweisungen durch eine Vorrichtung ausführbar sind, um zu bewirken, dass die Vorrichtung ein Verfahren durchführt, das Folgendes umfasst:
Erheben eines Bildes (130) eines Zielorgans eines Körpers (110) durch eine Ultraschallsonde (120; 210);
Verarbeiten des Bildes in Verbindung mit einem Schätzer (140; 220), der ein neuronales Netzwerk umfasst, wobei der Schätzer eine Abweichung (190) einer zeitgleichen Stellung, die eine aus dem Bild ersichtliche Ausrichtung der Ultraschallsonde angibt, von einer für die Bildgebung des Zielorgans (350) optimalen Stellung der Ultraschallsonde erzeugt;
wobei eine Stellungsänderung, die mit einer Trägheitsmesseinheit (250) erhalten wird, mit der mit dem Schätzer (140, 220) erhaltenen Abweichung unter Verwendung eines Kalman-Filters kombiniert wird, um die von dem Schätzer (140; 220) erzeugte Abweichung feinabzustimmen, und
Präsentieren der erzeugten Abweichung für einen Endbenutzer-Bediener der Ultraschallsonde.

13. Computerprogrammprodukt nach Anspruch 12, wobei Sondenausrichtungs- und - bewegungsdaten (180) von einer Trägheitsmesseinheit (250) empfangen werden, die mindestens eines von einem Beschleunigungsmesser (250C), einem Gyroskop (250B) und einem Magnetometer (250A) umfasst.

14. Computerprogrammprodukt nach Anspruch 12, wobei die erzeugte Abweichung (190) visuell auf einer Anzeige eines Computersystems, das mit der Sonde (120; 210) gekoppelt ist, präsentiert wird.

15. Computerprogrammprodukt nach Anspruch 12, wobei die erzeugte Abweichung (190) hörbar durch ein Variieren eines Tons basierend auf einer Nähe der Sonde (120; 210) zu der optimalen Stellung präsentiert wird.

16. Computerprogrammprodukt nach Anspruch 12, wobei die erzeugte Abweichung (190) hörbar durch Variieren einer Frequenz einer wiederholten hörbaren Präsentation eines kurzen Tons basierend auf einer Nähe der Sonde (120; 210) zu der optimalen Stellung präsentiert wird.

17. Computerprogrammprodukt nach Anspruch 12, wobei die erzeugte Abweichung haptisch durch ein Variieren von Vibrationen der Sonde (120; 210) basierend auf einer Nähe der Sonde zu der optimalen Stellung präsentiert wird.

## Revendications

1. Procédé d'assistance à la navigation par ultrasons comprenant les étapes consistant à :
acquérir une image (130) par une sonde à ultrasons (120 ; 210) d'un organe cible d'un corps (110) (330) ;
traiter l'image en liaison avec un estimateur (140 ; 220) comprenant un réseau neuronal, l'estimateur produisant un écart (190) d'une position contemporaine indiquant une orientation de la sonde à ultrasons évidente à partir de l'image par rapport à une position optimale de la sonde à ultrasons pour l'imagerie de l'organe cible (350) ;
dans lequel un changement de position obtenu avec une unité de mesure inertielle (250) est combiné à un écart produit par l'estimateur (140, 220) à l'aide d'un filtre de Kalman, pour ajuster l'écart produit par l'estimateur (140 ; 220) et,
présenter l'écart produit à un opérateur utilisateur final de la sonde à ultrasons.

2. Procédé de la revendication 1, dans lequel les données d'orientation et de mouvement de sonde (180) sont reçues de l'unité de mesure inertielle (250) comprenant au moins un accéléromètre (250C), un gyroscope (250B) et un magnétomètre (250A).

3. Procédé de la revendication 1, dans lequel l'écart produit (190) est présenté visuellement sur un écran d'un système informatique couplé à la sonde (120 ; 210).

4. Procédé de la revendication 1, dans lequel l'écart produit (190) est présenté de manière audible par la variation d'une tonalité basée sur la proximité de la sonde (120 ; 210) par rapport à la position optimale.

5. Procédé de la revendication 1, dans lequel l'écart produit (190) est présenté de manière audible en variant la fréquence de présentation répétée d'un son de courte durée en fonction de la proximité de la sonde (120 ; 210) par rapport à la position optimale.

6. Procédé de la revendication 1, dans lequel l'écart produit (190) est présenté de manière haptique par une variation des vibrations de la sonde en fonction de la proximité de la sonde (120 ; 210) par rapport à la position optimale.

7. Système de traitement des données d'imagerie par ultrasons configuré pour l'assistance à la navigation par ultrasons, le système comprenant :
un ordinateur doté d'une mémoire et d'au moins un processeur ;
un écran couplé à l'ordinateur ;
un circuit de formation de faisceau couplé à l'ordinateur et à l'écran ;
une sonde à ultrasons (120 ; 210) comprenant un transducteur connecté au circuit de formation de faisceau ; et,
un module d'assistance à la navigation (300) s'exécutant dans la mémoire de l'ordinateur, le module comprenant un code de programme activé lors de l'exécution par le processeur de l'ordinateur pour acquérir une image (130) par la sonde à ultrasons d'un organe cible d'un corps (110), pour traiter avec un estimateur (140 ; 220) comprenant un réseau neuronal, l'image acquise en déterminant un écart (190) entre une position contemporaine de la sonde à ultrasons par rapport à l'organe cible évident à partir de l'image acquise, et une position optimale de la sonde à ultrasons pour l'imagerie de l'organe cible,
dans lequel un changement de position obtenu avec une unité de mesure inertielle (250) est combiné à l'écart produit par l'estimateur (140 ; 220) à l'aide d'un filtre de Kalman, pour ajuster l'écart produit par l'estimateur (140 ; 220) ; et
présenter l'écart déterminé à l'opérateur utilisateur final de la sonde à ultrasons.

8. Système de la revendication 7, dans lequel les données d'orientation et de mouvement de sonde (180) sont reçues de l'unité de mesure inertielle (250) comprenant au moins un accéléromètre (250C), un gyroscope (250B) et un magnétomètre (250A).

9. Système de la revendication 7, dans lequel l'écart déterminé (190) est présenté visuellement sur un écran d'un système informatique couplé à la sonde (120, 210).

10. Système de la revendication 7, dans lequel l'écart déterminé (190) est présenté de manière audible par la variation d'une tonalité basée sur la proximité de la sonde (120 ; 210) par rapport à la position optimale.

11. Système de la revendication 7, dans lequel l'écart déterminé (190) est présenté de manière haptique par une variation des vibrations de la sonde (120 ; 210) en fonction de la proximité de la sonde par rapport à la position optimale.

12. Produit de programme informatique pour l'assistance à la navigation par ultrasons, le produit de programme informatique comprenant un support de stockage lisible par ordinateur ayant des instructions de programme incorporées, les instructions de programme exécutables par un dispositif pour faire en sorte que le dispositif exécute un procédé comprenant mes étapes consistant à :
acquérir une image (130) par une sonde à ultrasons (120 ; 210) d'un organe cible d'un corps (110) ;
traiter l'image en liaison avec un estimateur (140 ; 220) comprenant un réseau neuronal, l'estimateur produisant un écart (190) d'une position contemporaine indiquant une orientation de la sonde à ultrasons évidente à partir de l'image par rapport à une position optimale de la sonde à ultrasons pour l'imagerie de l'organe cible (350) ;
dans lequel un changement de position obtenu avec une unité de mesure inertielle (250) est combinée à l'écart obtenu avec l'estimateur (140 ; 220) à l'aide d'un filtre de Kalman, pour ajuster l'écart produit par l'estimateur (140 ; 220) et
présenter l'écart produit à un opérateur utilisateur final de la sonde à ultrasons.

13. Produit de programme informatique de la revendication 12, dans lequel les données d'orientation et de mouvement de la sonde (180) sont reçues d'une unité de mesure inertielle (250) comprenant au moins un accéléromètre (250C), un gyroscope (250B) et un magnétomètre (250A).

14. Produit de programme informatique de la revendication 12, dans lequel l'écart produit (190) est présenté visuellement sur un écran d'un système informatique couplé à la sonde (120 ; 210).

15. Produit de programme informatique de la revendication 12, dans lequel l'écart produit (190) est présentée de manière audible par la variation d'une tonalité en fonction de la proximité de la sonde (120 ; 210) par rapport à la position optimale.

16. Produit de programme informatique de la revendication 12, dans lequel l'écart produit (190) est présenté de manière audible en faisant varier la fréquence de présentation répétée d'un son de courte durée en fonction de la proximité de la sonde (120 ; 210) par rapport à la position optimale.

17. Produit de programme informatique de la revendication 12, dans lequel l'écart produit est présenté de manière haptique par une variation des vibrations de la sonde (120 ; 210) en fonction d'une proximité de la sonde par rapport à la position optimale.
